# EUROPEAN PATENT SPECIFICATION

(11) **EP 4 413 937 B1**
(45) Date of publication and mention of the grant of the patent: **28.10.2026**
(21) Application number: 24151144.3
(22) Date of filing: 10.01.2024
(51) Int. Cl.: A61B 18/20

(54) **SKIN TREATMENT DEVICE**
HAUTBEHANDLUNGSVORRICHTUNG
DISPOSITIF DE TRAITEMENT DE LA PEAU

(30) Priority: 08.02.2023 GB 202301777
(43) Date of publication of application: 14.08.2024
(60) Divisional application: 26199738.1 / 4 817 545
(73) Proprietor: iPulse Limited, Swansea, SA1 8QB (GB)
(72) Inventor: JONES, Stuart Terry, Swansea, SA1 8QB (GB)
(74) Representative: Murgitroyd & Company

(56) References cited:
- WO-A1-2022/195267
- US-A1- 2007 239 142
- US-A1- 2021 178 182
- US-A1- 2022 346 871

## Description

The present invention relates to a skin treatment device, preferably an Intense Pulsed Light (IPL) hair removal device for generation of high intensity pulses of broadband light.

Skin treatment devices by light are known for example from US2007/239142A1 or US2022/346871A1.

The light pulses are typically generated by discharging the electrical energy stored in a capacitor through a xenon flashlamp delivering light energy in the form of pulses. The light from the lamp is passed through a filter to remove potentially harmful wavelengths in the ultraviolet and blue region. The light is then directed onto an area of skin for treatment. The light energy that is emitted from the device and thus reaches the skin is called the 'light output energy'.

For an IPL device using a flashlamp, the conversion of stored electrical energy into light output energy generally has an efficiency of between 20% and 40%. The remaining 60% to 80% of the energy becomes waste heat generated directly by the lamp and electronics and within the components in the light path (e.g. light guides, filter) due to absorption (i.e. reflective or transmissive losses). Therefore: 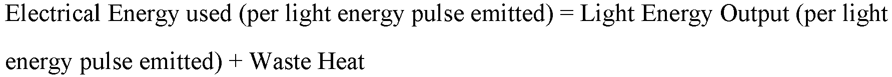 Electrical Energy used (per light energy pulse emitted) = Light Energy Output (per light energy pulse emitted) + Waste Heat

As an approximation, it will be assumed that an IPL device using a xenon flashlamp has an efficiency of 25%. That is, the ratio of Waste Heat / Light Energy Output is 3 / 1. Furthermore, Optical Power (in Watts) can be defined as: $Optical Power = Light Output Energy per flash / Time between flashes$

Therefore, for the example provided, the Waste Heat Power (in Watts) is: $Waste Heat Power = 3 * Optical Power$

It is generally beneficial for an IPL device to have a relatively high optical power. A higher optical power allows the device to deliver efficacious levels of energy per flash at a higher flash rate. This means that a device with higher optical power can treat a given area of skin more quickly and/or effectively than a device with lower optical power. This is particularly useful for body areas that have a large area to be treated such as legs, chest or back. From these power equations and the illustrative values, it can be understood that the higher the energy per flash, and/or the faster the device flash rate, the larger the amount of waste heat generated per second (waste heat power).

This creates a problem in IPL devices as the waste heat must be removed to prevent damage to the device itself or burn the user. This waste heat generated by the device is therefore typically removed by a forced air cooling system. This includes a small electric fan in the device that draws ambient air into the device, directing it over hot internal components (e.g. lamp, reflector etc.) before exhausting the air back out of the handset. The heat removal capacity of the forced air cooling system must be designed to match or exceed the waste heat power of the device to prevent overheating of the handset components.

Due to the requirement to ensure that the light energy pulses are actually delivered to the skin and that stray optical radiation should be minimised to avoid unsafe levels for eyes, the device should be in contact with the skin (or imperceptibly close). Stray optical radiation can be defined as light emitted by the light source that is intended to be absorbed by the target skin area for treatment purposes, but either misses the intended target or is reflected or remitted from the target. This has a problem in that waste heat will be transferred from the device to the skin. Accordingly, how the device contacts the skin must be carefully considered. There are two main types of skin contact configuration in existing devices.

### Type 1 - Non-contact - Hollow light pipe

The light energy pulse emitted by the lamp is filtered and then transmitted to the skin through a hollow 'light pipe' which is typically made from a thin reflective aluminium material wall defining a tube/channel which sits between the filter and the skin. The light pipe tube is hollow and therefore, the light energy pulses generated by the lamp pass through the air inside the light pipe until it hits the skin. The benefits of this arrangement are firstly aluminium light pipes are cheap to manufacture. Secondly, since the light passes through air to hit the skin, there is no solid medium in the direct light energy transfer pathway in contact with the skin to get hot due to transmission losses, and therefore no need for a complex cooling arrangement to ensure the skin contacting material remains cool.

Such Type 1 devices are capable of emitting relatively high optical power, typically in the order of 20W, meaning larger areas of the body such as legs or the back can be treated relatively quickly as the pulse repetition rate is high. Such Type 1 devices are less beneficial however for body areas that are small and more sensitive to heat such as underarms and bikini areas. Accordingly, Type 2 devices are available.

### Type 2 - Contact - Solid Light Guide

The light generated by the lamp is filtered and then transmitted to the skin through a solid light guide that contacts the skin when in operation. The light guide is typically made from a transparent material such as glass or sapphire.

The solid light guide material will heat up due to transmission losses and conduction during each discharge of a light energy pulse. If the solid light guide is not specifically cooled, it may eventually reach a temperature that is painful or harmful to the skin. Therefore, a solid light guide is typically used in combination with a thermoelectric cooling system (TEC) to extract heat from the light guide to keep it cool which can also act to provide a cooling effect to the skin below normal skin temperature. This TEC system adds additional cost, complexity, size and weight to the device. The thermoelectric device itself also creates additional waste heat which must be exhausted by the forced air cooling system.

The higher the optical power of the device, the higher the waste heat input to the solid light guide and therefore, the higher the TEC power required to keep the light guide cool, adding even greater levels of cost, bulk, and complexity to the device. Typical Type 2 devices emit a maximum optical power of 8W. The power, size and cost of the TEC system must increase proportionally to the optical power. Therefore, for a given optical power, a Type 2 device would be larger, heavier and more expensive than a Type 1 device. However, solid lightguides do have a significant benefit in that they may have a high transmission efficiency and importantly can be cooled to a temperature below normal skin temperature. This cools the surface of the skin being treated which makes the treatment safer and less painful. This is particularly helpful on more sensitive areas such as underarms and bikini areas.

It is therefore apparent that Type 1 (non-contact) devices are better suited to higher optical power devices making treatment fast for a given treatment fluence and are therefore better at treating larger body areas such as legs or backs. A Type 2 (contact) system is better suited to treatment of sensitive areas (such as underarms and bikini areas) as the sapphire light guide can be cooled to a temperature below skin temperature. This cools the surface of the skin during treatment making the treatment less painful.

It is notable that the most sensitive body areas to be treated (underarms and bikini area) are relatively small and therefore the lower power and speed of the Type 2 system for these areas is not a significant issue.

Multiple devices are therefore required to allow the beneficial effects of Type 1 and Type 2 treatment; however, this is impractical, expensive and mostly not viable, particularly for home-use applications.

The present invention aims to address the above-mentioned deficiencies or at least provide a useful alternative.

According to the present invention there is a skin treatment device for delivery of light energy pulses to a subject's skin as defined in claim 1.

The device therefore combines the benefits of a Type 1 system (high power, speed) in the second configuration and a Type 2 system (safety and low pain) in the first configuration, without a corresponding increase in size, cost and weight, and with a simple reconfiguration by a user.

With the head mounted onto the body, the head spaces the primary skin contact surface away from the skin thereby defining a secondary light guide between the primary skin contact surface and the second skin contact surface (defined by a leading edge of the head). Accordingly, with the head mounted to the body, the primary skin contact surface is rearwardly of the second skin contact surface.

The second skin contact surface of the head beneficially defines an output window. The second skin contact surface is preferably defined by a peripheral rim extending around an opening into the secondary lightguide.

It will be appreciated that in the mounted configuration a light energy transfer pathway from the light source to the forward end (and therefore onto the skin) is through the primary and secondary lightguides.

It will be appreciated that in the second configuration the secondary lightguide is defined by the head and light energy is guided from the skin contact surface of the solid material of the primary lightguide toward the skin.

The device is beneficially configured to detect whether the head is mounted or demounted from the housing. A detector is beneficially provided. The detector is beneficially the control system configured to determine communication with a microprocessor in the head.

The light energy pulse transmission channel preferably comprises a bore extending from the opening towards the rearward end. The bore may extend the entire way from the opening to the rearward end.

The cooling arrangement is beneficially a Thermoelectric Cooling System (TEC). The cooling system may be only operable in the first configuration due to the separation of the first skin contact surface from the skin in the second configuration meaning cooling of the first skin contact surface is unnecessary. The TEC preferably comprises a thermoelectric cooler, a heat exchanger and a heat pipe therebetween, the TEC further comprising a fan for directing air towards the heat exchanger.

The device is preferably configured to detect whether the head is mounted or demounted from the housing, and where the control system is configured to modify operation of the skin treatment device dependent on the whether the head is mounted or demounted from the housing.

The control system is configured to control delivery of multiple light energy pulses and to control delivery of an optical power output, the control system being further configured to cause modification to the optical power output dependent upon whether the head is mounted or demounted from the housing, where the optical power output is defined as light output energy per pulse emitted from the device divided by the time between consecutive pulses. The value of the optical power output is therefore beneficially modified. The device is preferably capable of modifying operation of the device without user input. This provides a safety benefit to the user. The control system can therefore modify how the device operates dependent upon whether the head is mounted to the body. The optical power output is greater when the head is mounted to the body, wherein the optical power may be between 1.5-3 times greater when the head is mounted to the body.

The control system is preferably arranged to control delivery of multiple light energy pulses, and the control system is further configured to control a time period between delivery of consecutive pulses and select the time period from a plurality of different time periods dependent upon whether the head is mounted or demounted from the body.

The time period is preferably less longer when the head is demounted from the housing. This means that with the head mounted to the housing, the light energy pulse repetition rate is higher. Accordingly, a user of the device will be able to treat larger body areas faster with the head mounted to the body.

It will also be appreciated that the optical power output can be modified by modifying the light output energy per pulse. Light energy pulses are typically emitted by discharging a capacitor over the light energy source. By modifying the voltage on the capacitor for example the light output energy emitted by the light source can be modified.

With the head mounted and thus in the second configuration a higher optical power output, for example of 20W results in a period between delivery of light energy pulses of 0.9 seconds when the total light energy output is 18J. With the head removed in the first configuration the optical power output is for example 6W, which would result in a period between delivery of light energy pulses of 3 seconds when the total light energy output is 18J. For these illustrative values, if the primary and secondary light guides have a cross sectional area of 3cm², then a total energy is output of 18J, giving a fluence of 6J/cm². A typical pulse duration for an IPL hair removal device with this fluence level is in the range 0.5msec to 2.5msec.

The device preferably further comprises a user input trigger switchable between a deactivated and activated configuration, the activated configuration for activating discharge of the light energy pulses, where the control system is arranged such that maintaining the user input trigger in an activated position causes discharge of multiple light energy pulses at a pulse repetition frequency. The device is therefore operable in different ways depending on a user input. If a user depresses and holds the user input trigger light energy pulses are emitted consecutively at the maximum permitted repetition rate controlled by the control system. Single pulses may also be emitted if the user input trigger is depressed and released where release occurs before the time period for a consecutive pulse to be emitted is reached. A consecutive pulse can then be emitted when the user input trigger is depressed again, providing that the time period has been met.

It will be understood that in the second configuration the primary and secondary lightguide combined define at least part of the pathway for light energy pulses from the light source to the skin of a subject.

It will be understood that the solid material of the primary lightguide can transmit light therethrough. The solid material of the primary lightguide preferably comprises glass or sapphire.

The light energy pulse transmission channel of the secondary lightguide preferably comprises a through-bore, where preferably the whole of the light energy pulse transmission channel is a through-bore. The light energy pulse transmission channel is preferably defined by a channel wall, where the channel comprises an airgap. The channel wall guides light energy pulses through the channel and is beneficially reflective. The secondary light guide is beneficially a hollow lightguide. An alternative equivalent terminology is that the secondary lightguide is a light pipe. The channel wall may comprise aluminium.

The material of the primary lightguide may not extend the entirety of the distance between the light source and the skin contact surface. Accordingly, there may be a gap between the light source and the material. The primary light guide preferably further comprises a filter for filtering out predetermined wavelengths of light energy. The filter preferably has a cut-on value in the range of 470 to 650 nm. The filter may comprise a coating on the solid material. Alternatively, the filter may comprise a distinct and separate component to the solid material intermediate the light source and the solid material.

The device preferably further comprises a temperature sensor arranged to measure the temperature of the solid material of the primary lightguide, and where the control system is arranged to control operating parameters of the cooling arrangement dependent upon the measured temperature. The control system is preferably arranged to reduce the optical power output if the measured temperature reaches or exceeds a threshold value. The control system may be configured to prevent delivery of a light energy pulse if the measured temperature reaches or exceeds a threshold value.

The cooling arrangement is preferably activated in both first and second operable configurations. The cooling arrangement may be operated in a reduced power mode in the second operable configuration.

The head preferably comprises a plurality of sensors disposed adjacent the opening, where in the second configuration the control system is arranged to receive sensor outputs from the plurality of sensors and based on the sensor outputs control operation of the device.

The body preferably comprises a plurality of sensors disposed adjacent the first skin contact surface where in the first configuration the control system is arranged to receive sensor outputs from the plurality of sensors and based on the sensor outputs control operation of the device. The plurality of sensors disposed adjacent the first skin contact surface are either hidden by the head or are inoperable in the in the second configuration with the head mounted to the body.

Controlling operation of the device may comprise one or both of determining whether or not the flashlamp can emit a light energy pulse and determining properties of that pulse (e.g. fluence).

The device may comprise an adaptor for securing to the body arranged to partially obscure the first skin contact surface. This enables ease of use for treatment of smaller body areas.

At least a portion of the forward end of the head comprising the second skin contact surface is preferably at least partially moveable from a rest position to a deflected position to accommodate the geometry of the skin.

The light source is beneficially a flashlamp, preferably a xenon flashlamp.

The skin treatment device is beneficially a handheld skin treatment device. The body is beneficially arranged to be grasped by a user.

Aspects of the present invention will now be described by way of illustration only with reference to the accompany Figures where:
Figure 1 is a schematic perspective view of a device according to an illustrative embodiment of the present invention with a head attached in Figure 1a and detached in Figure 1b.
Figure 2 is a schematic cross sectional view of a device according to an illustrative embodiment of the present invention with a head attached in Figure 2a and detached in Figure 2b.
Figure 3 is schematic perspective view of a device according to an illustrative embodiment of the present invention with a head attached in Figure 3a and detached in Figure 3b.
Figure 4 is schematic perspective view of a device according to an illustrative embodiment of the present invention with a head attached in Figure 4a and detached in Figure 4b.

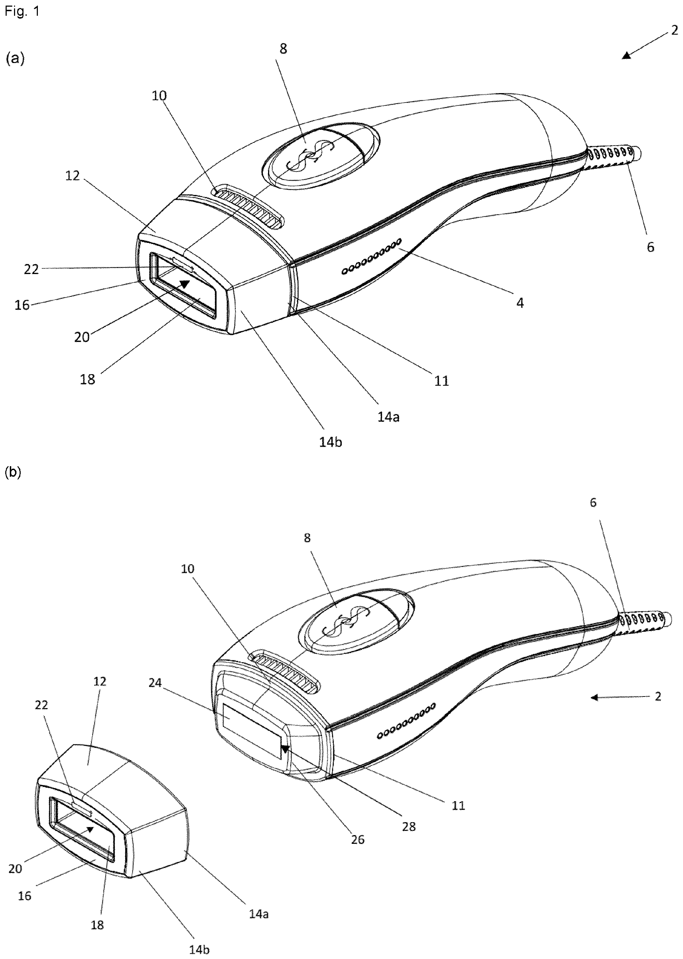

Referring to Figure 1a there is a schematic perspective representation of an illustrative embodiment of the present invention. Presented is an IPL device 2 comprising a housing/body 4 arranged to be received in the hand of a user. The device connects to a mains power source via cord 6 and comprises a user operated trigger in the form of a push button 8 for causing operation. A vent 10 is provided in the housing for exhausting hot air. At a forward end 11 of the housing 4 a head 12 is shown mounted relative to the housing 4. The head comprises a rearward end 14a arranged to mount and demount relative to the housing 4 and a forward end 14b defining a (second) skin contact surface 16 in the form of a peripheral rim surrounding an output window 18 through which light energy pulses are transmitted. The output window 18 is an opening into the head 12, specifically into a secondary lightguide 20 typically in the form of a channel comprising aluminium channel walls for directing the light energy pulses through the output window 18 and onto the skin of a user. The channel may comprise a through-bore extending through the entirety of the head from the forward to a rearward ends 14b,14a. One or more sensors are provided in the skin contact surface 16 for determining contact with the skin of a user, from which an output is transmitted to the control system for controlling operation of the device 2. With the head 12 mounted to the housing 4, the device is operable in a configuration where there is no physical contact of the leading end of the body 4 with the user's skin, rather it is the peripheral rim of the head that contacts the skin.

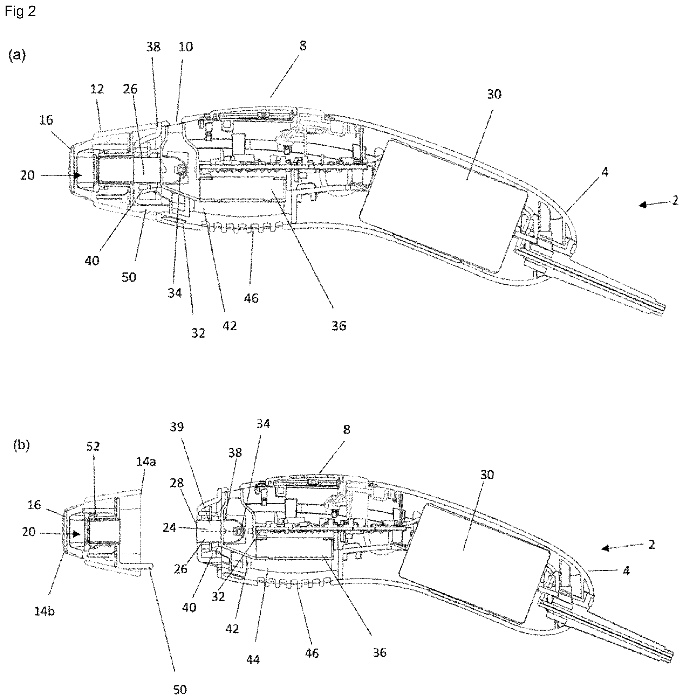

Referring now to Figure 1b, the head 12 is demounted from the housing 4. The device 2 is therefore operable in a first configuration without the presence of the head 12. The forward end of the housing 4 is shaped to receive the head 12 and comprises a primary lightguide 24 comprising a material 26 having a first skin contact surface 28. In operation, the first skin contact surface 28 of the material 26 communicates with the skin and provides a cooling effect as described further with respect to Figure 2.

Referring now to Figure 2 a cross sectional view of an illustrative embodiment of the present invention is presented with the head 12 in communication with the housing 4 (Figure 2a) and with the head 12 removed from the housing 4 (Figure 2b). The device comprises a charge storage device 30 in the form of a capacitor and a control system 32 for controlling operation of the device including discharge of the capacitor over the flashlamp 34. Further provided is a fan 36 for directing cooling air across the flashlamp 34 and out of the vent 10. Referring to Figure 2b, the primary lightguide represented by a dashed line 24 is defined between the flashlamp 34 and the first skin contact surface 28. The primary lightguide 24 comprises the solid material 26 typically comprising glass or sapphire having at a trailing edge a filter 38 which may be a coating on the material 26 for filtering most harmful wavelengths of light in the UV and blue wavelength ranges that are inherently emitted by the flashlamp 34. The primary lightguide comprises a light reflective wall defining a channel 39 in which is positioned the solid material 26. The solid material 26 abuts against the wall meaning light energy pulses must travel through the solid material.

During operation of the device in a first operable configuration with the head 12 demounted from the housing 4 the solid material 26 will heat up due to transmission losses and conduction during the emission of each energy pulse from the flashlamp 34. Without cooling the material 26 it is likely that the material may reach a temperature painful or harmful to the skin. The device 2 therefore further comprises a cooling arrangement for cooling the material 26 in the form of a Thermoelectric Cooling System (TEC) comprising a thermoelectric cooler such as a peltier 40, heat pipes 42 and heat exchanger 44. The fan 36 drives air across the heat exchanger 44. Air inlet 46 is provided in the housing for enabling passage of cooling air to the heat exchanger 44. A significant benefit of utilising a solid material 26 is that it may be cooled to a temperature below normal skin temperature which means the skin contact surface 28 of the material 26 cools the skin which is particularly beneficial for sensitive treatment areas such as underarm and bikini areas.

The rearward end 14a of the head 12 is arranged to be mounted to the leading portion of the housing 4 through a connector 50. In the illustrative embodiment the connector 50 extends outwardly from the rearward end 14a of the head 12 and seats into a corresponding opening in the forward end of the housing 4. Engagement between the connector 50 and the housing couples the head 12 to the housing 4. Additional or alternative connections may be provided for enabling engagement such as mechanical or magnetic attachment mechanisms. Electronics present in the head (as described further below) are coupled to the control system with the head mounted by the connector 50.

The device is capable of determining that the head is mounted to the body and/or that the head is detached from the body and operation of the device is modified. This may be achieved for example by a sensor output or the detection of an electrical connection between electronics provided in the head. The operative parameter is typically power, and the control system enables a higher output power output with the head mounted compared to the optical power output with the head demounted. In the first configuration with the head detached, as an example the optical power is at a reduced value of 6W. To produce this power the optical power may be output at 18J and 3 seconds between flashes, where 18J may be 6J/cm² for a 3cm² treatment area. The TEC is operational at a suitable power to control the temperature of the solid material 26 such that the skin contact surface 28 cools the skin. In the second operative configuration with the head mounted to the housing, a relatively high optical power can be output. For example, 20W may be produced by 18J and 0.9 seconds between flashes, where 18J may again be 6J/cm² for a 3cm² treatment area. In this second operational configuration, the TEC may be switched off or operated at reduced power (compared to the optical power) due to the spacing of the first skin contact surface 28 from the skin.

Referring back to Figure 2, the head 12 defines the second skin contact surface 16 which sits against the skin in use. The secondary lightguide is defined between the first skin contact surface 28 and the second skin contact surface 16. The secondary lightguide comprises a reflective wall 52 defining a channel or pathway for the light energy pulses. The channel is an airgap, and the secondary lightguide may be termed a light pipe. Typical lightguides comprise a reflective aluminium wall defining the channel, with no solid medium that can contact the skin. The second skin contact surface is the peripheral rim of the leading end of the head 12.

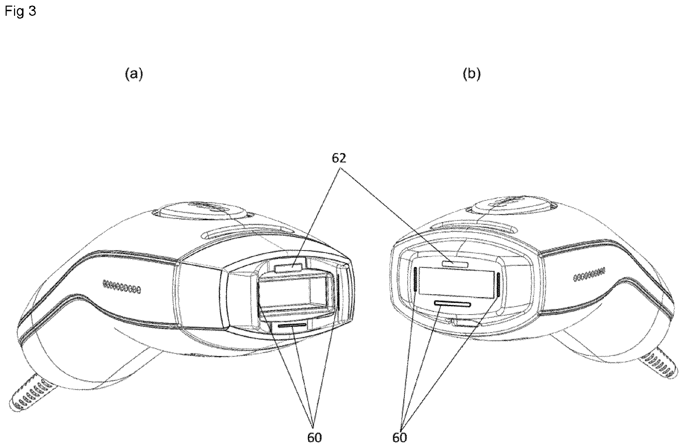

Referring now to Figure 3a and b a perspective representation of a device according to an illustrative embodiment of the invention is presented from a front view with the head 12 attached (Figure 3a) and detached (Figure 3b). It is important in devices according to the present invention that good skin contact is required before allowing emission of a pulse of energy from the flashlamp. Good contact can be defined as a condition where the light output area is sufficiently covered by skin that any stray optical radiation is below harmful levels. As such, safety features are implemented so that the device will not emit radiation unless the device is in contact with a user's skin to minimise stray optical radiation from the device in operation which may be at unsafe levels for the eyes. This is typically achieved through the provision of multiple sensors adjacent each side of the output window (for example above, below and to either side of a rectangular output window) in the head of the device where a surface must be detected by each sensor as a requirement for radiation to be emitted. If one sensor does not measure a threshold value, then it is determined by the control system of the device that there is no skin contact and firing is prevented. This is to prevent the device firing when good contact with the skin is not achieved with the associated risk of the emission of potentially harmful levels of stray optical radiation. Stray optical radiation can be defined as light emitted by the device that is intended to be absorbed by the target skin area for treatment purposes, but either misses the intended target, is reflected or remitted from the target. Various sensors may be utilised such as capacitive contact sensors or proximity sensors.

As shown in Figure 3a and b in either condition (with or without the head attached) there are multiple capacitive skin contact sensors 60 positioned around the peripheral edge of the exit of the first and second lightguides. The sensors 60 are positioned around the peripheral rim 16 of the head 14 are disposed around the skin contact surface 28 of the material 26 with the head 14 removed. Outputs from the sensors are received by the control system, and control of the device is dependent upon the sensor output. For example, assuming a threshold capacitance is measured from the sensors, then the control system in the device does not prevent emission of an energy pulse as it is deemed there is contact with the skin and emission of an energy pulse is safe. An additional sensor such as a skin tone sensor 62 (which may measure reflectance) may be provided and the control system may use this measurement both to control whether an energy pulse may be emitted and also be used to control the energy of the light energy pulse emitted dependent upon the reflectance (and therefore determined skin tone).

In an embodiment of the invention, the sensors 60/62 at the forward end of the housing that are described as being disposed around the peripheral edge of the output window are carried by a detachable cover, arranged to connect to the control system and operate in the same way as described above.

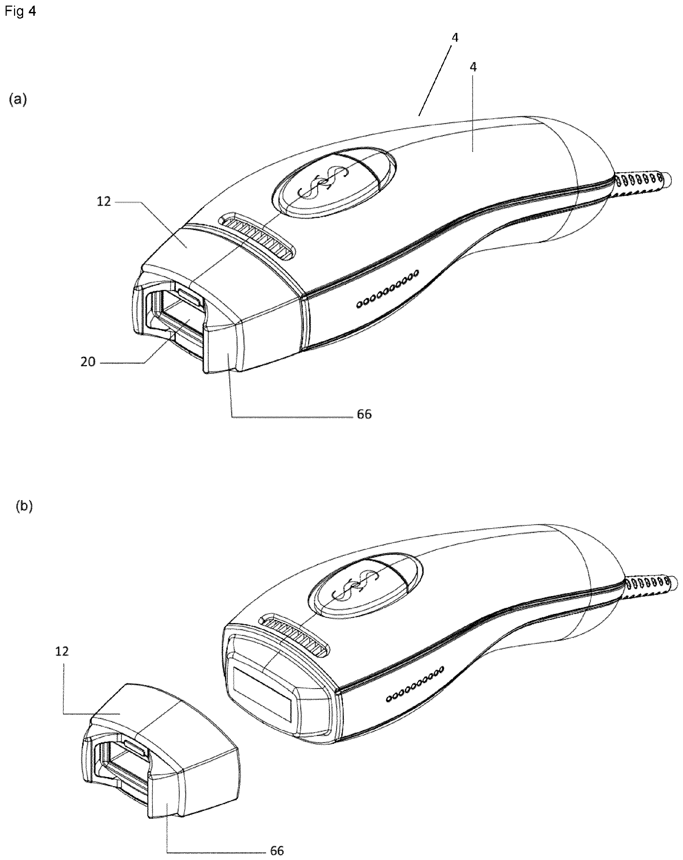

Referring now to Figure 4a and b, there is a schematic perspective view of a device according to an illustrative embodiment of the present invention with a head attached in Figure 4a and detached in Figure 4b. In this illustrative embodiment (and as schematically also presented in Figure 3a) at least a portion of the forward end of the head 12 may be moveable. The forward end of the head 12 comprises a second skin contact surface 16. At least a part of the forward end of the head having the skin contact surface may be at least partially moveable from a rest position to a deflected position in order to accommodate the geometry of the skin to be treated. This may be achieved through the provision for example of one or more projections (two shown in the illustrative embodiment) 66 arranged to deflect independently of one another to conform to the geometry of the skin. Each of the projections carry a sensor 60/62. The or each projection 66 is arranged to be received into a corresponding opening in the body of the head 12, where the projection in the rest position extends outwardly from the opening in the head body, and in the deflected position the projection is at least partially withdrawn into an opening in the head body. The at least one projection is beneficially biased to the rest position by a spring and are each pivotally mounted relative to the head 12 body.

The device according to any of the illustrative embodiments may be arranged to monitor the temperature of the material 26 of the primary lightguide 24, and the control system may be arranged to control operating parameters of the TEC dependent upon the measured values. A suitable temperature sensor may be utilised such as a thermocouple of resistive temperature measuring device. The control system 32 may be configured to control parameters of the TEC (such as the peltier current) to maintain a constant material temperature at the first skin contact surface 28 independent of the speed in which the user activates the device to effect emission of consecutive pulses. The temperature monitoring may also be used to control the optical power (through either modification of the time between discharge of consecutive light energy pulses or energy per pulse) to assist with reduction of waste heat generated in the device and allow the material 26 to cool further and faster. Additionally, the temperature monitoring may also be utilised such that the control system prevents emission of a light energy pulse unless the material 26 temperature is within a predetermined temperature range. This means that if the temperature is too high for safe treatment, then discharge of a light energy pulse is prevented. This may occur when the device is first switched on, or when transitioning from the second to the first operational configuration, particularly if the device was operating at high optical power in the second configuration.

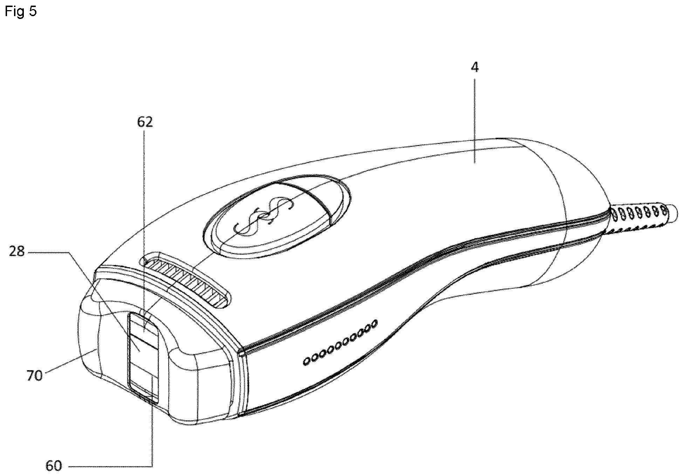

Additional optional features according to illustrative embodiment of the present invention may be the provision of multiple different heads 12 with different cross-sectional areas of the light output window. Furthermore, as shown in Figure 5 an adaptor 70 may be secured to the forward end of the housing 4 arranged to reduce the first skin contact surface area 28. In such an embodiment the treatment area is reduced meaning that small areas, such as top lip can be treated with improved usability. Sensors 60 that are covered by the adaptor 70 are beneficially disabled in such a configuration.

It will be appreciated that in each of the illustrative embodiment the device is operable in at least two configurations, where the head 12 is either mounted to the housing 4 or is demounted from the housing 4. Accordingly, a significant advantage is provided in that a single device is reconfigurable depending on the area of the body in which the user wishes to treat. Therefore, the device 2 combines high power and speed of a Type 1 system with a increased safety and reduced pain of a Type 2 system in a single device without a corresponding increase in size, cost and weight.

Aspects of the present invention have been described by way of illustration only and it will be understood by a skilled addressee that modifications and variations may be made without departing from the scope of protection afforded by the appended claims.

## Claims

1. A skin treatment device (2) for delivery of light energy pulses to a subject's skin, the skin treatment device comprising:
- a body (4) ;
- a light source (34) housed within the body for discharging light energy pulses;
- a control system (32) for controlling delivery of the light energy pulses;
- a primary light guide (24) for guiding light energy from the light source to the skin of the subject, the primary light guide comprising a solid material through which the light energy pulses are transmitted, the solid material defining a first skin contact surface (28) for providing contact with a subject's skin;
- a cooling arrangement for cooling the solid material;
- a head (12) arranged to be mountable and demountable to the body, the head comprising a rearward end for releasably mounting to the body and extending to a forward end comprising a second skin contact surface, the head further comprising a secondary lightguide defining a light energy pulse transmission channel extending between the forward end and rearward end with the second skin contact surface defining an opening to the light energy pulse transmission channel, where the second skin contact surface extends around at least a part of the periphery of the opening;
the device being operable in a first configuration with the head demounted from the body and a second configuration where the head is mounted to the body such that in the second configuration the first skin contact surface is spaced apart from the subject's skin and the light energy pulses pass through both the solid material of the first lightguide and the light energy pulse transmission channel of the second lightguide; wherein
the control system is configured to control delivery of multiple light energy pulses and to control delivery of an optical power output, the control system being further configured to cause modification to the optical power output dependent upon whether the head is mounted or demounted from the housing, where the optical power output is defined as light output energy per pulse emitted from the device divided by the time between consecutive pulses, and **characterized in that**
the optical power output is greater when the head is mounted to the body.

2. A skin treatment device according to claim 1 where the device is configured to detect whether the head is mounted or demounted from the housing, and where the control system is configured to modify operation of the skin treatment device dependent on the whether the head is mounted or demounted from the housing.

3. A skin treatment device according to claim 2 wherein the optical power is between 1.5-3 times greater when the head is mounted to the body.

4. A skin treatment device according to claim 1 where the control system is arranged to control delivery of multiple light energy pulses, and the control system is further configured to control a time period between delivery of consecutive pulses and select the time period from a plurality of different time periods dependent upon whether the head is mounted or demounted from the body.

5. A skin treatment device according to claim 4 wherein the time period is longer when the head is demounted from the body.

6. A skin treatment device according to claim 2 further comprising a detector for detecting whether the head is mounted or demounted from the body.

7. A skin treatment device according to claim 1 where the solid material of the primary lightguide comprises glass or sapphire.

8. A skin treatment device according to claim 1 wherein the primary light guide further comprises a filter for filtering out predetermined wavelengths of light energy; and/or wherein a majority of the light energy pulse transmission channel comprises a through-bore.

9. A device according to claim 1 where the second skin contact surface is defined by a peripheral rim extending around the opening to the light energy pulse transmission channel of the secondary lightguide.

10. A device according to claim 1 where the cooling arrangement is a Thermoelectric Cooling System, TEC, and optionally wherein the TEC comprises a thermoelectric cooler, a heat exchanger and a heat pipe therebetween, the TEC further comprising a fan for directing air towards the heat exchanger.

11. A device according to claim 1 comprising a temperature sensor arranged to measure the temperature of the solid material of the primary lightguide, and where the control system is arranged to control operating parameters of the cooling arrangement dependent upon the measured temperature; and optionally where the control system is arranged to reduce the optical power output if the measured temperature reaches or exceeds a threshold value; and optionally where the control system is configured to prevent delivery of a light energy pulse if the measured temperature reaches or exceeds a threshold value.

12. A device according to claim 1 wherein the cooling arrangement is activated in both the first and second operable configurations; and/or wherein the head comprises a plurality of sensors disposed adjacent the opening, where in the second configuration the control system is arranged to receive sensor outputs from the plurality of sensors and based on the sensor outputs control operation of the device; and/or wherein the body comprises a plurality of sensors disposed adjacent the first skin contact surface where in the first configuration the control system is arranged to receive sensor outputs from the plurality of sensors and based on the sensor outputs control operation of the device.

13. A device according to claim 1 further comprising an adaptor for securing to the body arranged to partially obscure the first skin contact surface; and/or wherein at least a portion of the forward end of the head comprising the second skin contact surface is at least partially moveable from a rest position to a deflected position to accommodate the geometry of the skin.

## Patentansprüche

1. Eine Hautbehandlungsvorrichtung (2) zur Abgabe von Lichtenergieimpulsen an die Haut eines Individuums, wobei die Hautbehandlungsvorrichtung Folgendes beinhaltet:
- einen Körper (4);
- eine Lichtquelle (34), die innerhalb des Körpers untergebracht ist, um Lichtenergieimpulse auszustoßen;
- ein Steuersystem (32) zum Steuern der Abgabe der Lichtenergieimpulse;
- einen primären Lichtleiter (24) zum Leiten von Lichtenergie von der Lichtquelle zu der Haut des Individuums, wobei der primäre Lichtleiter ein festes Material beinhaltet, durch das die Lichtenergieimpulse übertragen werden, wobei das feste Material eine erste Hautkontaktfläche (28) zum Bereitstellen von Kontakt mit der Haut eines Individuums definiert;
- eine Kühlanordnung zum Kühlen des festen Materials;
- einen Kopf (12), der so angeordnet ist, dass er an dem Körper montierbar und von dem Körper demontierbar ist, wobei der Kopf ein hinteres Ende zum lösbaren Montieren an dem Körper beinhaltet und sich zu einem vorderen Ende erstreckt, das eine zweite Hautkontaktfläche beinhaltet, wobei der Kopf ferner einen sekundären Lichtleiter beinhaltet, der einen Lichtenergieimpulsübertragungskanal definiert, der sich zwischen dem vorderen Ende und dem hinteren Ende erstreckt, wobei die zweite Hautkontaktfläche eine Öffnung zu dem Lichtenergieimpulsübertragungskanal definiert, wobei sich die zweite Hautkontaktfläche um mindestens einen Teil des Umfangs der Öffnung erstreckt;
wobei die Vorrichtung in einer ersten Konfiguration, in der der Kopf von dem Körper demontiert ist, und einer zweiten Konfiguration, in der der Kopf so an dem Körper montiert ist, dass die erste Hautkontaktfläche in der zweiten Konfiguration von der Haut des Individuums beabstandet ist und die Lichtenergieimpulse sowohl durch das feste Material des ersten Lichtleiters als auch den Lichtenergieimpulsübertragungskanal des zweiten Lichtleiters verlaufen, betrieben werden kann;
wobei das Steuersystem so konfiguriert ist, dass es die Abgabe mehrerer Lichtenergieimpulse steuert und die Abgabe einer optischen Leistungsabgabe steuert, wobei das Steuersystem ferner so konfiguriert ist, dass es eine Änderung der optischen Leistungsabgabe in Abhängigkeit davon bewirkt, ob der Kopf an dem Gehäuse montiert oder von dem Gehäuse demontiert ist, wobei die optische Leistungsabgabe als von der Vorrichtung emittierte Lichtabgabeenergie pro Impuls, dividiert durch die Zeit zwischen aufeinanderfolgenden Impulsen, definiert ist, und **dadurch gekennzeichnet, dass** die optische Leistungsabgabe größer ist, wenn der Kopf an dem Körper montiert ist.

2. Hautbehandlungsvorrichtung gemäß Anspruch 1, wobei die Vorrichtung so konfiguriert ist, dass sie erkennt, ob der Kopf an dem Gehäuse montiert oder von dem Gehäuse demontiert ist, und wobei das Steuersystem so konfiguriert ist, dass es den Betrieb der Hautbehandlungsvorrichtung in Abhängigkeit davon ändert, ob der Kopf an dem Gehäuse montiert oder von dem Gehäuse demontiert ist.

3. Hautbehandlungsvorrichtung gemäß Anspruch 2, wobei die optische Leistung um das 1,5- bis 3-Fache größer ist, wenn der Kopf an dem Körper montiert ist.

4. Hautbehandlungsvorrichtung gemäß Anspruch 1, wobei das Steuersystem so angeordnet ist, dass es die Abgabe mehrerer Lichtenergieimpulse steuert, und das Steuersystem ferner so konfiguriert ist, dass es einen Zeitraum zwischen der Abgabe aufeinanderfolgender Impulse steuert und den Zeitraum aus einer Vielzahl von unterschiedlichen Zeiträumen in Abhängigkeit davon auswählt, ob der Kopf an dem Körper montiert oder von dem Körper demontiert ist.

5. Hautbehandlungsvorrichtung gemäß Anspruch 4, wobei der Zeitraum länger ist, wenn der Kopf von dem Körper demontiert ist.

6. Hautbehandlungsvorrichtung gemäß Anspruch 2, die ferner einen Detektor zum Erkennen, ob der Kopf an dem Körper montiert oder von dem Körper demontiert ist, beinhaltet.

7. Hautbehandlungsvorrichtung gemäß Anspruch 1, wobei das feste Material des primären Lichtleiters Glas oder Saphir beinhaltet.

8. Hautbehandlungsvorrichtung gemäß Anspruch 1, wobei der primäre Lichtleiter ferner einen Filter zum Herausfiltern vorbestimmter Wellenlängen von Lichtenergie beinhaltet; und/oder wobei ein Großteil des Lichtenergieimpulsübertragungskanals eine Durchgangsbohrung beinhaltet.

9. Vorrichtung gemäß Anspruch 1, wobei die zweite Hautkontaktfläche durch einen Umfangsrand definiert ist, der sich um die Öffnung zum Lichtenergieimpulsübertragungskanal des sekundären Lichtleiters erstreckt.

10. Vorrichtung gemäß Anspruch 1, wobei die Kühlanordnung ein thermoelektrisches Kühlsystem, TEC (Thermoelectric Cooling), ist,
und optional wobei das TEC einen thermoelektrischen Kühler, einen Wärmetauscher und ein Wärmerohr dazwischen beinhaltet, wobei das TEC ferner ein Gebläse zum Leiten von Luft in Richtung des Wärmetauschers beinhaltet.

11. Vorrichtung gemäß Anspruch 1, die ferner einen Temperatursensor beinhaltet, der so angeordnet ist, dass er die Temperatur des festen Materials des primären Lichtleiters misst, und wobei das Steuersystem so angeordnet ist, dass es Betriebsparameter der Kühlanordnung in Abhängigkeit von der gemessenen Temperatur steuert; und optional wobei das Steuersystem so angeordnet ist, dass es die optische Leistungsabgabe reduziert, wenn die gemessene Temperatur einen Schwellenwert erreicht oder überschreitet; und optional wobei das Steuersystem so konfiguriert ist, dass es die Abgabe eines Lichtenergieimpulses verhindert, wenn die gemessene Temperatur einen Schwellenwert erreicht oder überschreitet.

12. Vorrichtung gemäß Anspruch 1, wobei die Kühlanordnung sowohl in der ersten als auch in der zweiten betreibbaren Konfiguration aktiviert wird; und/oder wobei der Kopf eine Vielzahl von Sensoren beinhaltet, die angrenzend an die Öffnung angeordnet sind, wobei das Steuersystem in der zweiten Konfiguration so angeordnet ist, dass es Sensorausgaben von der Vielzahl von Sensoren empfängt und basierend auf den Sensorausgaben den Betrieb der Vorrichtung steuert; und/oder wobei der Körper eine Vielzahl von Sensoren beinhaltet, die angrenzend an die erste Hautkontaktfläche angeordnet sind, wobei das Steuersystem in der ersten Konfiguration so angeordnet ist, dass es Sensorausgaben von der Vielzahl von Sensoren empfängt und basierend auf den Sensorausgaben den Betrieb der Vorrichtung steuert.

13. Vorrichtung gemäß Anspruch 1, die ferner einen Adapter zum Befestigen an dem Körper beinhaltet, der so angeordnet ist, dass er die erste Hautkontaktfläche teilweise verdeckt; und/oder wobei mindestens ein Abschnitt des vorderen Endes des Kopfs, der die zweite Hautkontaktfläche beinhaltet, mindestens teilweise aus einer Ruheposition in eine ausgelenkte Position beweglich ist, um sich an die Geometrie der Haut anzupassen.

## Revendications

1. Un dispositif de traitement de la peau (2) destiné à administrer des impulsions d'énergie lumineuse à la peau d'un sujet, le dispositif de traitement de la peau comprenant :
- un corps (4) ;
- une source lumineuse (34) logée à l'intérieur du corps et destinée à émettre des impulsions d'énergie lumineuse ;
- un système de commande (32) destiné à commander l'administration des impulsions d'énergie lumineuse ;
- un guide de lumière primaire (24) destiné à guider l'énergie lumineuse de la source lumineuse jusqu'à la peau du sujet, le guide de lumière primaire comprenant un matériau solide à travers lequel les impulsions d'énergie lumineuse sont transmises, le matériau solide définissant une première surface de contact avec la peau (28) destinée à assurer le contact avec la peau d'un sujet ;
- un agencement de refroidissement destiné à refroidir le matériau solide ;
- une tête (12) agencée de façon à pouvoir être montée sur le corps et démontée de celui-ci, la tête comprenant une extrémité arrière destinée à être montée de façon amovible sur le corps et s'étendant jusqu'à une extrémité avant comprenant une deuxième surface de contact avec la peau, la tête comprenant en outre un guide de lumière secondaire définissant un canal de transmission d'impulsions d'énergie lumineuse s'étendant entre l'extrémité avant et l'extrémité arrière, la deuxième surface de contact avec la peau définissant une ouverture sur le canal de transmission d'impulsions d'énergie lumineuse, où la deuxième surface de contact avec la peau s'étend autour d'au moins une partie de la périphérie de l'ouverture ;
le dispositif pouvant fonctionner dans une première configuration dans laquelle la tête est démontée du corps et une deuxième configuration dans laquelle la tête est montée sur le corps de telle sorte que, dans la deuxième configuration, la première surface de contact avec la peau est espacée de la peau du sujet et que les impulsions d'énergie lumineuse passent à travers à la fois le matériau solide du premier guide de lumière et le canal de transmission d'impulsions d'énergie lumineuse du deuxième guide de lumière ; dans lequel le système de commande est configuré de façon à commander l'administration de multiples impulsions d'énergie lumineuse et à commander l'administration d'une sortie de puissance optique,
le système de commande étant configuré en outre de façon à provoquer une modification de la sortie de puissance optique selon que la tête est montée sur le boîtier ou démontée de celui-ci, où la sortie de puissance optique est définie comme étant l'énergie de sortie lumineuse par impulsion émise à partir du dispositif divisée par le temps entre des impulsions consécutives, et **caractérisé en ce que** la sortie de puissance optique est plus importante lorsque la tête est montée sur le corps.

2. Un dispositif de traitement de la peau selon la revendication 1 où le dispositif est configuré de façon à détecter si la tête est montée sur le boîtier ou démontée de celui-ci, et où le système de commande est configuré de façon à modifier le fonctionnement du dispositif de traitement de la peau selon que la tête est montée sur le boîtier ou démontée de celui-ci.

3. Un dispositif de traitement de la peau selon la revendication 2 dans lequel la puissance optique est entre 1,5 et 3 fois plus importante lorsque la tête est montée sur le corps.

4. Un dispositif de traitement de la peau selon la revendication 1 où le système de commande est agencé de façon à commander l'administration de multiples impulsions d'énergie lumineuse, et le système de commande est configuré en outre de façon à commander une période de temps entre l'administration d'impulsions consécutives et à sélectionner la période de temps parmi une pluralité de périodes de temps différentes selon que la tête est montée sur le corps ou démontée de celui-ci.

5. Un dispositif de traitement de la peau selon la revendication 4 dans lequel la période de temps est plus longue lorsque la tête est démontée du corps.

6. Un dispositif de traitement de la peau selon la revendication 2 comprenant en outre un détecteur destiné à détecter si la tête est montée sur le corps ou démontée de celui-ci.

7. Un dispositif de traitement de la peau selon la revendication 1 où le matériau solide du guide de lumière primaire comprend du verre ou du saphir.

8. Un dispositif de traitement de la peau selon la revendication 1 dans lequel le guide de lumière primaire comprend en outre un filtre destiné à éliminer par filtrage des longueurs d'onde prédéterminées d'énergie lumineuse ; et/ou dans lequel une majorité du canal de transmission d'impulsions d'énergie lumineuse comprend un alésage traversant.

9. Un dispositif selon la revendication 1 où la deuxième surface de contact avec la peau est définie par un rebord périphérique s'étendant autour de l'ouverture jusqu'au canal de transmission d'impulsions d'énergie lumineuse du guide de lumière secondaire.

10. Un dispositif selon la revendication 1 où l'agencement de refroidissement est un système de refroidissement thermoélectrique, TEC,
et facultativement dans lequel le TEC comprend un refroidisseur thermoélectrique, un échangeur de chaleur et un caloduc entre eux, le TEC comprenant en outre un ventilateur destiné à diriger de l'air vers l'échangeur de chaleur.

11. Un dispositif selon la revendication 1 comprenant un capteur de température agencé de façon à mesurer la température du matériau solide du guide de lumière primaire, et où le système de commande est agencé de façon à commander des paramètres de fonctionnement de l'agencement de refroidissement en fonction de la température mesurée ; et facultativement où le système de commande est agencé de façon à réduire la sortie de puissance optique si la température mesurée atteint ou dépasse une valeur seuil ; et facultativement où le système de commande est configuré de façon à empêcher l'administration d'une impulsion d'énergie lumineuse si la température mesurée atteint ou dépasse une valeur seuil.

12. Un dispositif selon la revendication 1 dans lequel l'agencement de refroidissement est activé à la fois dans la première et la deuxième configuration de fonctionnement possible ; et/ou dans lequel la tête comprend une pluralité de capteurs disposés de façon adjacente à l'ouverture, où, dans la deuxième configuration, le système de commande est agencé de façon à recevoir des sorties de capteur provenant de la pluralité de capteurs et, en fonction des sorties de capteur, à commander le fonctionnement du dispositif ; et/ou dans lequel le corps comprend une pluralité de capteurs disposés de façon adjacente à la première surface de contact avec la peau où, dans la première configuration, le système de commande est agencé de façon à recevoir des sorties de capteur provenant de la pluralité de capteurs et, en fonction des sorties de capteur, à commander le fonctionnement du dispositif.

13. Un dispositif selon la revendication 1 comprenant en outre un adaptateur destiné à être fixé au corps agencé de façon à masquer partiellement la première surface de contact avec la peau ; et/ou dans lequel au moins une portion de l'extrémité avant de la tête comprenant la deuxième surface de contact avec la peau est au moins partiellement déplaçable d'une position de repos à une position déviée de façon à s'adapter à la géométrie de la peau.
